# EUROPEAN PATENT SPECIFICATION

(11) **EP 3 799 445 B1**
(45) Date of publication and mention of the grant of the patent: **25.06.2025**
(21) Application number: 20198762.5
(22) Date of filing: 28.09.2020
(51) Int. Cl.: H04R 25/00, A61B 5/00, A61B 5/0205, A61B 5/024, A61B 5/0537

(54) **HEARING SYSTEM INCLUDING A SENSOR ELECTRODE AND METHOD FOR MANUFACTURING THE SAME**
HÖRSYSTEM MIT EINER SENSORELEKTRODE UND VERFAHREN ZUR HERSTELLUNG DAVON
SYSTÈME AUDITIF COMPRENANT UNE ÉLECTRODE CAPTEUR ET SON PROCÉDÉ DE FABRICATION

(30) Priority: 30.09.2019 US 201916587739
(43) Date of publication of application: 31.03.2021
(73) Proprietor: Sonova AG, 8712 Stäfa (CH)
(72) Inventor: MÜLLER, Markus, 8708 Männedorf (CH); SILBERZAHN, Konstantin, 8706 Meilen (CH); THUMM, Natasha, 8620 Wetzikon (CH)

(56) References cited:
- WO-A1-2020/142154
- JP-A- H09 122 083
- US-A1- 2015 023 539
- US-A1- 2019 111 261
- US-A1- 2019 117 155

## Description

### BACKGROUND INFORMATION

Hearing systems are used to improve the hearing capability and/or communication capability of users. Such hearing systems are configured to process a received input sound signal (e.g., ambient sound) and then provide the processed input sound signal to the user (e.g., through a hearing device such as a hearing aid). Hearing systems may use an in-the-ear ("ITE") component to facilitate providing the processed input sound signal to the user. Such ITE components are configured to fit at least partially within an ear canal of the user.

In addition to being used to facilitate providing the processed input sound signal to the user, such ITE components may also include electrodes that may be configured to contact ear tissue within the ear canal of the user while the ITE component is worn by the user. Such electrodes may be used, for example, to detect electrical signals within tissue of the user. However, such electrodes may be difficult to manufacture due to space constraints within the ITE component. Moreover, to contact the ear tissue within the user's ear canal, the electrodes typically protrude from the outer surface of an ITE component. As such, the electrodes may make the ITE component itchy, painful, and/or generally uncomfortable for the user to wear.
US 2019/0111261 A1 relates to an electrode for a hearing device that electrically couples to the skin of a user. The electrode may include an outer conductor and an inner conductor which individually can be sub-flush, flush or super-flush to an outer surface of a housing of the hearing device. The electrode may be coupled to other hearing device components by using wire conductors.
JP H09-122083 A describes a patient monitoring device that can be worn at all times in the ear canal of a patient and which includes one or more sensors that detect biometric data. The sensors are connected to a control and transmission unit via lead wires for transmission of the data.
US 2019/0117155 A1 describes an ear-worn device as e.g. a hearing aid including one or more temperature sensors that can be implemented as thermistors. The sensors can be integrated as part of a shell and can protrude into the skin of the ear. The sensors are connected to a flexible or rigid circuit on the inside of the shell. The shell comprises a faceplate which can also bear a temperature sensor.
US 2015/023539 A1 relates to hearing assistance systems and more particularly to methods and apparatus for embedded conductive traces for hearing assistance device housings.

### SUMMARY

It is an object of the present disclosure to avoid at least one of the above mentioned disadvantages and to facilitate manufacturing of the ITE component and/or to provide the ITE component with advantageous properties for the wearing inside a user's ear and/or for accommodating components inside.

At least one of these objects can be achieved by a hearing system comprising the features of patent claim 1**.** Advantageous embodiments of the invention are defined by the dependent claims and the following description.

Accordingly, the present disclosure proposes a hearing system configured to assist a user in hearing, wherein the hearing system comprises an in-the-ear (ITE) component comprising: a shell having a contoured outer shape that is customized for the user to fit at least partially within an ear canal of the user; a faceplate configured to fit within an opening provided in the shell and face out of the ear canal of the user when the shell is inserted into the ear canal of the user; a sensor electrode that is provided on an outer surface of the shell; and a conductive path that extends along the outer surface of the shell from the sensor electrode to the faceplate and that conductively connects the sensor electrode with a conductive portion of the faceplate.

Independently, the present disclosure proposes a method of manufacturing an in-the-ear (ITE) component of a hearing system configured to assist a user in hearing, the method comprising: forming a pattern on an outer surface of a shell having a contoured outer shape that is customized for the user to fit at least partially within an ear canal of the user, the pattern including a sensor electrode pattern and a conductive path pattern; adding conductive material to the sensor electrode pattern and the conductive path pattern to form a sensor electrode and a conductive path; and attaching a faceplate to the shell such that the conductive path extends along the outer surface of the shell from the sensor electrode to the faceplate and conductively connects the sensor electrode with a conductive portion of the faceplate.

Independently, the present disclosure proposes a sensor system for use with an in-the-ear (ITE) component of a hearing system configured to assist a user in hearing, the sensor system comprising: a sensor electrode provided on an outer surface of a shell of the ITE component, the shell having a contoured outer shape that is customized for the user to fit at least partially within an ear canal of the user; circuitry configured to use the sensor electrode to detect a physiological attribute of the user; and a conductive path that extends along the outer surface of the shell from the sensor electrode to a faceplate of the ITE component and that conductively connects the sensor electrode to a conductive portion on the faceplate.

Subsequently, additional features of some implementations of the hearing system and/or the method and/or the sensor system are described. Each of those features can be provided solely or in combination with at least another feature. The features can be correspondingly provided in some implementations of the hearing system and/or the method and/or the sensor system.

It may be that the shell is a three-dimensional printed shell that is custom printed for the user to fit at least partially within the ear canal of the user. Accordingly, the method may further comprise three-dimensionally printing the shell such that the shell is customized for the user to fit at least partially within the ear canal of the user.

It may be that the shell is formed of a material that includes an additive for laser direct structuring (LDS); and the sensor electrode and the conductive path are patterned on the outer surface of the shell by an LDS process that activates the additive for LDS. Accordingly, in the method, the forming of the pattern on the outer surface of the shell may include using a laser direct structuring (LDS) process to form the pattern and activate an additive for LDS included within the shell.

In some implementations, the faceplate is an injection molded faceplate that is formed of a thermoplastic material that includes an additive for laser direct structuring (LDS). In some implementations, the faceplate is a three-dimensional printed faceplate that is custom printed for the user to fit the contoured outer shape of the shell. It may be that the conductive portion of the faceplate is conductively connected to circuitry housed within the shell.

In some implementations, the hearing system further comprises a behind-the-ear (BTE) component that is communicatively coupled to the ITE component and that includes circuitry configured to control operation of the sensor electrode, wherein the ITE component further comprises a receiver configured to acoustically deliver an audio signal to the user as directed by the BTE component.

In some implementations, the ITE component further comprises: a microphone configured to detect an audio signal; and a receiver configured to deliver the audio signal to the user.

In some implementations, the sensor electrode is configured to detect a physiological attribute of the user.

In some implementations, the conductive path is conductively connected to the conductive portion of the faceplate by at least one of conductive glue, soldering, spring contacts, and bolts with press-fit.

In some implementations, the ITE component further comprises: an additional sensor electrode provided on the outer surface of the shell; and an additional conductive path that extends along the outer surface of the shell from the additional sensor electrode to the faceplate and that conductively connects the additional sensor electrode with an additional conductive portion of the faceplate.

The hearing system and/or the sensor system may comprise circuitry configured to use the sensor electrode to detect a physiological attribute of the user. In some implementations, the circuitry is housed within the shell of the ITE component; and the conductive portion of the faceplate is conductively connected to the circuitry housed within the shell. In some implementations, the circuitry is housed within a behind-the-ear (BTE) component that is communicatively coupled to the ITE component; and the ITE component further comprises a receiver configured to acoustically deliver an audio signal to the user as directed by the BTE component.

In some implementations, the physiological attribute comprises at least one of a hydration level within the ear canal of the user, brain activity indicated in an electroencephalogram (EEG) measurement, and a heartbeat attribute indicated in an electrocardiogram (ECG) measurement.

### BRIEF DESCRIPTION OF THE DRAWINGS

The accompanying drawings illustrate various embodiments and are a part of the specification. The illustrated embodiments are merely examples and do not limit the scope of the disclosure. Throughout the drawings, identical or similar reference numbers designate identical or similar elements.
FIG. 1 illustrates an exemplary hearing system according to principles described herein.
FIG. 2 illustrates an exemplary ITE component of the hearing system shown in FIG. 1 according to principles described herein.
FIG. 3 illustrates an exemplary process for forming a sensor electrode and a conductive path on an outer surface of an ITE component according to principles described herein.
FIG. 4 illustrates an exemplary cross section of the ITE component shown in FIG. 2 that is taken along lines 4-4 in FIG. 2 according to principles described herein.
FIG. 5 illustrates an exemplary ITE component that includes a plurality of sensor electrodes according to principles described herein.
FIGS. 6 and 7 illustrate additional exemplary configurations of ITE components that may be implemented according to principles described herein.
FIG. 8 illustrates an exemplary sensor system according to principles described herein.
FIG. 9 illustrates an exemplary method for manufacturing a sensor electrode according to principles described herein.

### DETAILED DESCRIPTION

Hearing systems and sensor systems including a sensor electrode and methods for manufacturing the same are described herein. As will be described in more detail below, an exemplary hearing system configured to assist a user in hearing may include an ITE component that may comprise a shell having a contoured outer shape that is customized for the user to fit at least partially within an ear canal of the user, a faceplate configured to fit within an opening provided in the shell and face out of the ear canal of the user when the shell is inserted into the ear canal of the user, a sensor electrode that is provided on an outer surface of the shell, and a conductive path that extends along the outer surface of the shell from the sensor electrode to the faceplate and that conductively connects the sensor electrode with a conductive portion of the faceplate.

By providing a sensor electrode and a conductive path on an outer surface of a custom formed shell according to principles described herein, it is possible to provide an ITE component that is easier to manufacture and more comfortable for a user to wear than conventional ITE components that include electrodes. In addition, methods of manufacturing sensor electrodes such as those described herein may be more cost effective than methods of manufacturing conventional ITE components. Moreover, by manufacturing sensor electrodes and conductive paths according to principles described herein, it is possible to use space within ITE components more efficiently as compared to conventional ITE components. Other benefits of the hearing systems, sensor systems, and methods such as those described herein will be made apparent herein.

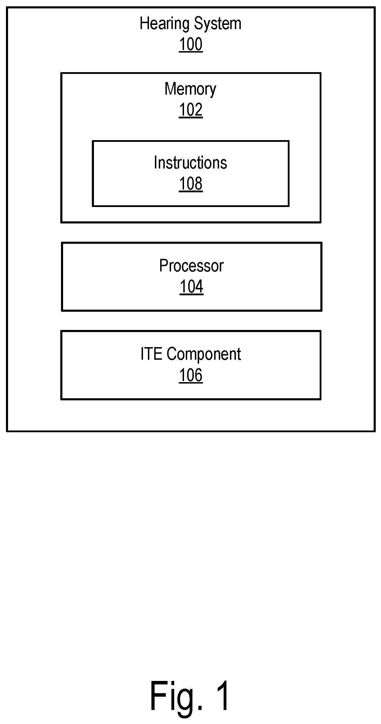

FIG. 1 illustrates an exemplary hearing system 100 that is configured to assist a user in hearing. As shown, hearing system 100 may include, without limitation, a memory 102, a processor 104, and an ITE component 106 selectively and communicatively coupled to one another. Memory 102 and processor 104 may each include or be implemented by hardware and/or software components (e.g., processors, memories, communication interfaces, instructions stored in memory for execution by the processors, etc.). In some examples, memory 102 and processor 104 may be housed within or form part of ITE component 106. In some examples, memory 102 and processor 104 may be located separately from ITE component 106 (e.g., in a behind-the-ear ("BTE") component). In some alternative examples, memory 102 and processor 104 may be distributed between multiple devices (e.g., multiple hearing devices in a binaural hearing system) and/or multiple locations as may serve a particular implementation.

Memory 102 may maintain (e.g., store) executable data used by processor 104 to perform any of the operations associated with hearing system 100 described herein. For example, memory 102 may store instructions 108 that may be executed by processor 104 to perform any of the operations associated with hearing system 100 described herein. Instructions 108 may be implemented by any suitable application, software, code, and/or other executable data instance.

Memory 102 may also maintain any data received, generated, managed, used, and/or transmitted by processor 104. For example, memory 102 may maintain any suitable data associated with physiological attributes of a user that may be detected using one or more sensor electrodes such as those described herein. As used herein, a "physiological attribute" may refer to any characteristic that may be associated with the functioning of the body of the user of hearing system 100. For example, a physiological attribute may comprise a hydration level within the ear canal of the user, brain activity indicated in an electroencephalogram ("EEG") measurement, a heartbeat attribute indicated in an electrocardiogram ("ECG") measurement, and/or any other suitable physiological attribute. Memory 102 may maintain additional or alternative data in other implementations.

Processor 104 is configured to perform any suitable processing operation that may be associated with hearing system 100. For example, when hearing system 100 includes a hearing aid device, such processing operations may include monitoring ambient sound and/or representing sound to a user via an in-ear receiver. In examples where hearing system 100 is included as part of a cochlear implant system, such processing operations may include directing a cochlear implant to generate and apply electrical stimulation representative of one or more audio signals (e.g., one or more audio signals detected by a microphone, input by way of an auxiliary audio input port, etc.) to one or more stimulation sites associated with an auditory pathway (e.g., the auditory nerve) of a recipient. Processor 104 may be implemented by any suitable combination of hardware and software.

In addition, processor 104 is configured to perform any suitable processing operation associated with hearing system 100 using one or more sensor electrodes such as those described herein to detect one or more physiological attributes of a user.

ITE component 106 is configured to facilitate hearing system 100 (e.g., processor 104) detecting such physiological attributes of the user. To that end, ITE component 106 is configured to fit at least partially within an ear canal of a user and may include a shell, a faceplate, a sensor electrode, and a conductive path. Each of these elements will now be described in detail. Various configurations of ITE component 106 including these elements will then be described in connection with the figures.

The shell of ITE component 106 is configured to fit at least partially within an ear canal of the user and has a contoured outer shape that is customized for a user. The shell may correspond to any suitable type of shell that may be used in hearing system 100 and that has a contoured outer shape that is custom made for a particular user.

The shell may be formed of any suitable material as may serve a particular implementation. For example, the shell may be formed of any one or a combination polymers such as Nylon, acrylonitrile butadiene styrene ("ABS"), polycarbonate ("PC"), polyphenylene sulfide ("PPS"), polyetheretherketone ("PEEK"), liquid crystal polymer, acrylics, and/or any other suitable material.

The shell may be manufactured using any suitable manufacturing process as may serve a particular implementation. In certain examples, a three-dimensional ("3D") printing process may be used to customize the shell to fit a particular user. By using a 3D printing process to manufacture a custom shell for a particular user, it is possible to reduce costs associated with manufacturing a custom shell as compared with other manufacturing methods such as injection molding. However, in certain alternative implementations, an injection molding process may be used to manufacture a custom shell to fit a particular user.

In examples where a shell is manufactured using a 3D printing process, 3D printable filaments that are amalgamated with an additive for laser direct structuring ("LDS") may be used to form the shell. The additive used for LDS is configured to facilitate forming a sensor electrode and a conductive path on an outer surface of the shell during an LDS process. Any suitable additive may be used for LDS as may serve a particular implementation. For example, an additive used for LDS may include a metal oxide (e.g., tin oxide), an organometallic complex (e.g., a palladium/palladium-containing heavy metal complex), and/or any other suitable material. Exemplary operations associated with using an LDS process to form a sensor electrode and conductive path on an outer surface of a shell will be described herein.

In certain examples, the shell may be a molded interconnect device ("MID") shell. MIDs are circuits that are directly integrated into the shape of a polymer component. An MID shell may be manufactured in any suitable manner. For example, an MID shell may be manufactured using an injection molding process in which any suitable material, such as those described herein, is injected into a mold to form the shape of the shell. The material injected into the mold may be amalgamated with an additive used for LDS, such as those described herein, to facilitate forming a sensor electrode and a conductive path on an outer surface of the shell during an LDS process.

The shell includes an opening that is configured to receive the faceplate to close the shell at a side oriented towards the exterior of the user's ear. The opening of the shell may have any suitable size and/or shape as may serve a particular implementation. In certain examples, the opening of the shell may have a standard size that is the same regardless of the particular shape of the ear canal of the user. In other implementations, the opening may have a custom shape that may be different for each particular user depending on the contoured outer shape of the shell.

The faceplate is configured to fit within the opening provided in the shell and face out of the ear canal of the user when the shell is inserted into the ear canal of the user. The faceplate may be formed of any suitable material. For example, the faceplate may be formed of the same material as the shell (e.g., Nylon, ABS, PC, PPS, PEEK, liquid crystal polymer, acrylics, etc.) or a different material.

The faceplate may be manufactured in any suitable manner. For example, in certain implementations, the faceplate may be 3D printed. In such examples, 3D printable filaments may be used to additively form the faceplate during a 3D printing process. In certain examples, the 3D printable filaments may be amalgamated with any suitable additive for LDS that may be used to form a conductive trace on a surface of the faceplate. Such a conductive trace may be configured to electrically connect the conductive path on the shell to electronics of the faceplate and/or electronics within the shell.

In certain alternative examples, the faceplate may be an injection molded faceplate formed of any suitable material for injection molding. For example, an injection molded faceplate may be formed of a thermoplastic material. Any suitable thermoplastic material may be used as may serve a particular implementation. For example, acrylics may be used to injection mold a faceplate in certain implementations. In certain examples, the material used to injection mold the faceplate may include an additive for LDS, such as those described herein. In such examples, the faceplate may be injection molded and then post processed as an MID in any suitable manner.

The faceplate may have any suitable configuration as may serve a particular implementation. For example, in certain implementations, the faceplate may have a standard shape that is configured to fit within a standard opening provided in the shell. Such a faceplate may be configured to fit within the standard opening in any suitable manner. For example, a faceplate with a standard shape may be clicked into the opening provided in the shell or may be glued into the opening. In such examples, the faceplate may be formed by cutting the faceplate to fit within the standard opening provided within the shell. Alternatively, the faceplate may be formed using an injection molding process.

In certain alternative implementations, the faceplate may be custom made to fit the contoured outer shape of the shell. For example, the faceplate may be cut in any suitable manner to fit the contoured outer shape of the shell. Alternatively, a 3D printing process may be used to manufacture the faceplate.

In certain examples, the faceplate may include one or more elements that facilitate operation and/or manual adjustment of hearing system 100. For example, in certain implementations, the faceplate may include a push button (e.g., that may be used to change hearing aid programs), a volume adjustment dial, a battery door usable to access a battery housed within the shell, and/or any other suitable element. In certain alternative implementations, the faceplate may not include any elements that facilitate manual adjustment of system 100. Rather, the faceplate may simply cover the opening in the shell and facilitate communicatively connecting the sensor electrode to circuitry within the shell or within another component such as a BTE component.

Additionally or alternatively, the faceplate may include one or more microphones, a vent to aid in the reduction of occlusion, a removal handle, and/or any other suitable feature.

At least a portion of the faceplate is configured to be conductive such that the conductive path connects the sensor electrode to a conductive portion of the faceplate. The conductive portion of the faceplate conductively connects the faceplate to any suitable circuitry or electronics that may be provided within the shell and/or outside of the shell (e.g., in a BTE component). The conductive portion of the faceplate may be formed in any suitable manner. For example, at least a portion of the faceplate may be provided with any suitable conductive particles.

Alternatively, a conductive trace may be formed on a portion of the faceplate in any suitable manner. For example, a conductive trace on the faceplate may be formed through an LDS process similar to that described herein with respect to the conductive path and sensor electrode on the outer surface of the shell. Such a conductive trace may be formed of any suitable metal or combination of metals. For example, the conductive trace may be formed of copper, nickel, gold, and/or any other suitable metal or combination of metals. In certain examples, the conductive portion of the faceplate may electrically connect to a printed circuit board ("PCB") provided on the faceplate.

In certain examples, the faceplate may include additional or alternative components to facilitate connecting electronics of the faceplate and/or electronics within the shell to the conductive path and the sensor electrode on the outer surface of the shell. For example, the faceplate may include inlays (e.g., metal rods, frames, etc.) or a PCB that may be directly integrated into the faceplate. Such features may be integrated into the faceplate in any suitable manner. For example, such features may be integrated into the faceplate during an injection molding process and/or an overmolding process. Alternatively, in examples where the faceplate has a standard shape, such features may be attached to the faceplate (e.g., by gluing) after the faceplate is formed.

According to the invention, the faceplate includes a rim configured to overlap the outer surface of the shell when the faceplate is inserted within the opening in the shell. In this case, the conductive portion of the faceplate is included on the rim such that, when the faceplate is inserted into the opening in the shell, the conductive portion electrically connects circuitry of hearing system 100 (e.g., within the shell) to the sensor electrode by way of the conductive path.

The sensor electrode is configured to be provided on an outer surface of the shell and is manufactured so as to follow the contoured outer shape of the shell. The sensor electrode may be used to detect any suitable information associated with the user. For example, the sensor electrode may be used to detect one or more physiological attributes of the user, such as those described herein.

The sensor electrode and the conductive path may be manufactured in any suitable manner. For example, the sensor electrode and conductive path may be manufactured through an LDS process, such as will be described herein. Alternatively, the sensor electrode and the conductive path may be formed through either an aerosol jet printing process or a ProtoPaint LDS process in certain implementations.

The sensor electrode and the conductive path may be formed of any suitable metal or combination of metals. For example, the sensor electrode may be formed of copper, nickel, gold, and/or any other suitable metal or combination of metals.

Any suitable number of sensor electrodes may be provided on the outer surface of the shell as may serve a particular implementation. For example, in certain implementations, only one sensor electrode may be provided on the outer surface of the shell. Alternatively, a plurality of sensor electrodes may be arranged in any suitable manner on the outer surface of the shell.

The sensor electrode may have any suitable, configuration, size, and/or shape as may serve a particular implementation. For example, the sensor electrode may be shaped as a square, a rectangle, a circle, etc. In certain examples, the sensor electrode may have a specific layout configured to facilitate detecting a particular type of physiological attribute of the user. For example, the sensor electrode may include a plurality of parallel electrode strips configured to create an electromagnetic field that may be used to determine skin capacitance of the user.

The sensor electrode may be positioned in any suitable location on the outer surface of the shell such that the sensor electrode is in direct contact with tissue within the ear canal when ITE component 106 is inserted within the ear canal. In certain examples, the sensor electrode may be located within a sealing zone associated with ITE component 106. As used herein, a "sealing zone" refers to a region around an outer circumference of the shell that has the acoustic function to seal the ear canal when ITE component 106 is worn by the user. It is understood that the outer surface of the shell that is in the sealing zone is in direct contact with the ear tissue in the ear canal when the shell is provided within the ear canal. As such, the sensor electrode may be positioned in any suitable location within the sealing zone to ensure that the sensor electrode is in direct contact with ear tissue of the user while ITE 106 component is worn by the user.

The conductive path is configured to electrically connect the sensor electrode to the faceplate. The conductive path is provided on the outer surface of the shell and, similar to the sensor electrode, is configured to follow the curvature of the outer surface of the shell. The conductive path may have any suitable thickness on the outer surface and/or may have any suitable length as may serve a particular implementation. In addition, the conductive path may take any suitable route from the sensor electrode to the faceplate. In certain examples, the conductive path may be covered with a thin protective coating made of any suitable material to protect and/or insulate the conductive path.

The electrical connection between the conductive path and the conductive portion of the faceplate may be facilitated in any suitable manner. For example, in certain implementations, the faceplate may overlap the shell when inserted within the opening in the shell such that the conductive portion of the faceplate is in direct contact with the conductive path on the outer surface of the shell. Additionally or alternatively, the electrical connection between the conductive path and the faceplate may be facilitated by using conductive glue, soldering, spring contacts, bolts with press-fit, and/or in any other suitable manner. In so doing, it may be possible to easily electrically connect the conductive path to circuitry on the faceplate, in the shell, and/or outside of the shell by way of the faceplate.

ITE component 106 may have any other suitable components as may serve a particular implementation. For example, in certain implementations, ITE component 106 may include a microphone configured to detect an audio signal. Additionally or alternatively, ITE component 106 may include a receiver (e.g., a speaker configured to deliver an audio signal to the user.

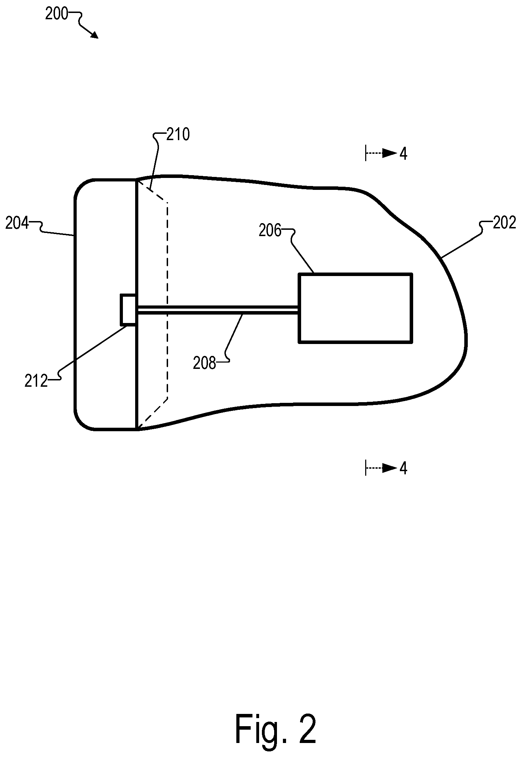

FIG. 2 shows an exemplary configuration 200 of ITE component 106. As shown in FIG. 2, ITE component 106 includes a shell 202, a faceplate 204, a sensor electrode 206, and a conductive path 208, each of which may be implemented as described above. As shown in FIG. 2, shell 202 has a contoured outer shape that is customized for a user to fit at least partially within an ear canal of the user. Faceplate 204 is configured to fit within an opening 210 of shell 202. In the example shown in FIG. 2, opening 210 of shell 202 is indicated by dashed lines and opens to the left to receive faceplate 204 and close shell 202 at a side oriented towards the exterior of the user's ear.

Sensor electrode 206 is provided on an outer surface of shell 202 at a position such that, when inserted within the ear canal, sensor electrode 206 may be in direct contact with ear tissue of the user within the ear canal.

Conductive path 208 conductively connects sensor electrode 206 to faceplate 204. As shown in FIG. 2, conductive path 208 extends along the outer surface of shell 202 from sensor electrode 206 to a conductive portion 212 of faceplate 204.

In the example shown in FIG. 2, conductive portion 212 is depicted as having a rectangular shape for illustrative purposes. However, it is understood that conductive portion 212 may be configured in any suitable manner, such as described herein. For example, conduct portion 212 may have any suitable shape and/or size as may serve a particular implementation. In addition, in certain examples, conductive portion 212 may be visible, as shown in FIG. 2. Alternatively, conductive portion 212 may not be visually discernable from other portions of faceplate 204 in certain implementations.

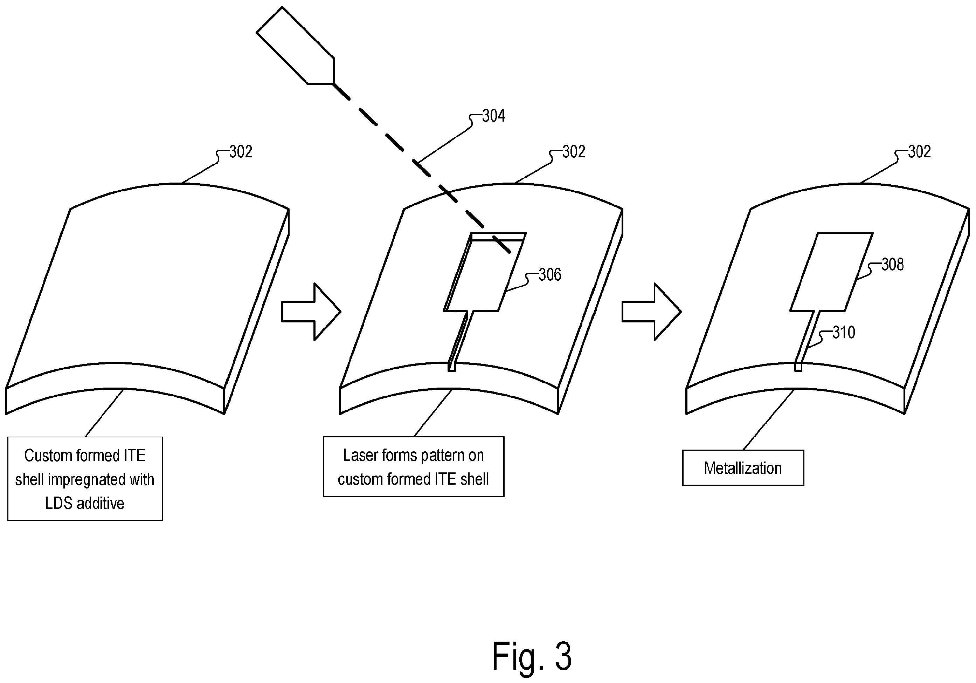

Sensor electrode 206 and conductive path 208 are formed such that they follow the contoured outer surface of shell 202. This is beneficial in that it results in ITE component 106 being more comfortable to wear than conventional ITE components. To manufacture sensor electrode 206 and conductive path 208 so that they have such characteristics, an LDS process may be used in certain implementations. Various operations that may performed during an LDS process will now be described with reference to FIG. 3.

As shown in FIG. 3, the leftmost image corresponds to a portion 302 of the outer surface of a custom formed ITE component shell (e.g., shell 202 of ITE component 106) that is amalgamated with an LDS additive (e.g., an organometallic additive). Although only portion 302 is shown in FIG. 3, it is understood that the LDS process shown in FIG. 3 would be performed on the whole custom formed ITE component shell (e.g., shell 202).

In the middle image of the LDS process shown in FIG. 3, an activation process is performed in which a computer-controlled laser beam 304 travels over portion 302 to expose a pattern 306 for the sensor electrode and the conductive path and activate the LDS additive. This activation process causes a physical-chemical reaction that produces metallic nuclei to which metal will attach during a metallization process. In addition to activating the LDS additive, laser beam 304 may form a microrough surface on which the metal can attach during the metallization process.

In the rightmost image shown in FIG. 3, a metallization process has been performed to form a sensor electrode 308 and a conductive path 310. Such a metallization process may be performed in any suitable manner. For example, the metallization process may include an additive conductor build-up performed in electroless copper baths. In certain examples, a plurality of different metals may be sequentially added to form sensor electrode 308 and conductive path 310. For example, electroless copper plating may be used to form a first metal layer formed of copper on the pattern. Afterwards, electroless nickel plating may be used to form a second metal layer of nickel on the second metal layer formed of copper. In certain examples, an electroless gold plating may also be used to form a third metal layer formed of gold on the second metal layer formed of nickel. Other types and/or combinations of metals may be built-up during the metallization process in other implementations.

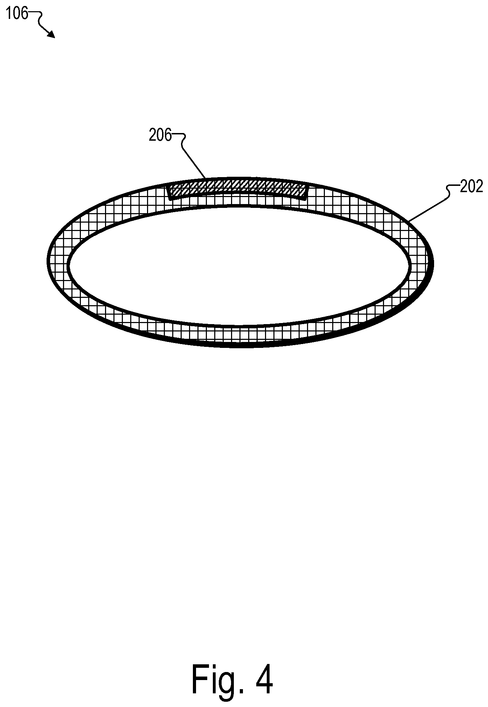

By using the LDS process described above, it is possible to manufacture sensor electrodes and conductive paths that follow the contoured outer shape of a shell and, as a result, are more comfortable when in contact with ear tissue of a user within the ear canal. To illustrate, FIG. 4 shows a cross-section of shell 202 shown in FIG. 2 that is taken along lines 4-4 in FIG. 2. As shown in FIG. 4, sensor electrode 206 has a contour that follows the contoured shape of the outer surface of shell 202 and is flush with the outer surface. With such a configuration, pressure from sensor electrode 206 is distributed evenly when shell 202 is worn by the user. As such, it is possible to mitigate harm and/or discomfort to the user.

In FIG. 4, the cross-sectional thickness of sensor electrode 206 is exaggerated for illustrative purposes. It is understood that the cross-sectional thickness of sensor electrode 206 may be relatively much less than the cross-sectional thickness of the wall of shell 202. For example, sensor electrode 202 may have a cross-sectional thickness on the order of a few microns whereas the wall of shell 202 may have a cross-sectional thickness on the order of a few millimeters. In addition, for simplicity, FIG. 4 shows the space within shell 202 as being empty. However, it is understood that the interior space may include any suitable circuitry, electronics, and/or other structures in certain implementations.

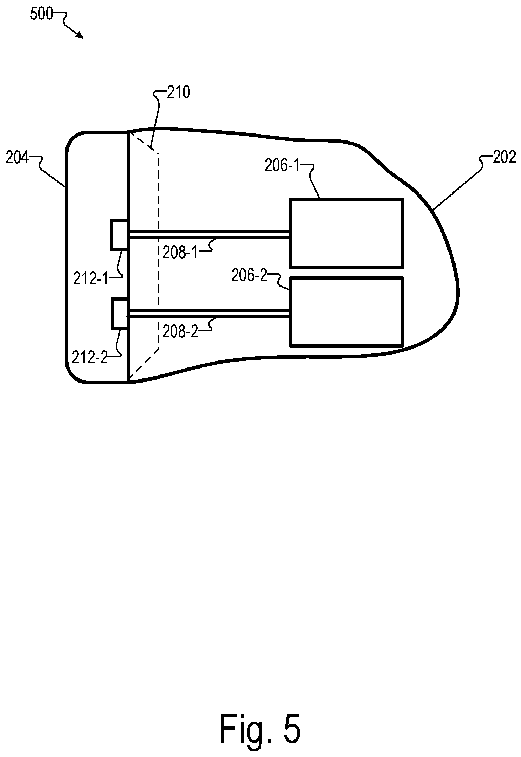

In certain examples, an additional sensor electrode may be provided on an outer surface of a shell. Such an additional sensor electrode may be electrically connected to a faceplate by way of an additional conductive path that extends along the outer surface of the shell from the additional sensor electrode to the faceplate. To illustrate, FIG. 5 shows an exemplary configuration 500 of ITE component 106 that includes two sensor electrodes 206 (e.g., sensor electrodes 206-1 and 206-2) that are each respectively electrically connected to conductive portions 212 (e.g., conductive portions 212-1 and 212-2) on faceplate 204 by way of conductive paths 208 (e.g., conductive paths 208-1 and 208-2). Although FIG. 5 shows sensor electrode 206-1 as being adjacent to sensor electrode 206-2, it is understood that sensor electrodes 206 could be arranged in any suitable manner with respect to one another in other implementations. For example, sensor electrode 206-1 may be provided on one side of shell 202 and sensor electrode 206-2 may be provided on an opposite side of shell 202 in certain implementations.

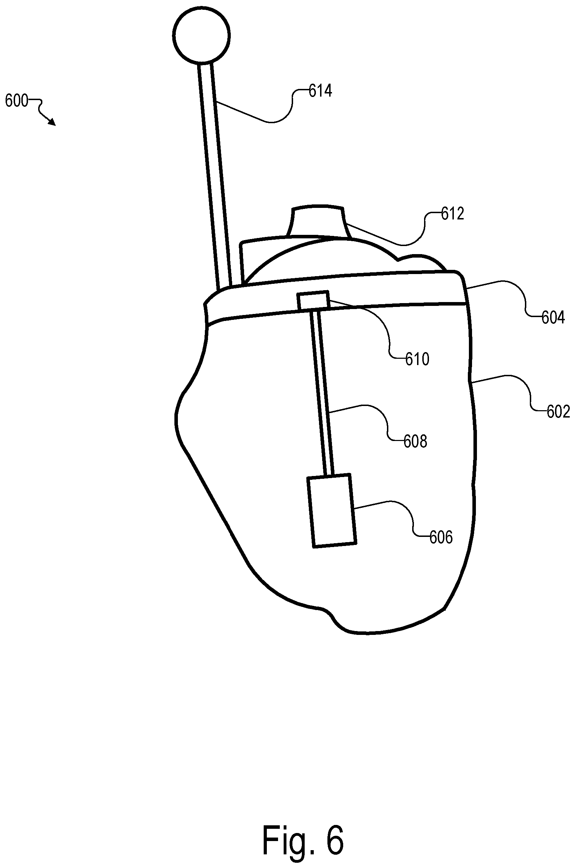

FIG. 6 shows an exemplary configuration 600 of ITE component 106 that may be provided in certain implementations when ITE component 106 corresponds to a hearing aid device. As shown in FIG. 6, configuration 600 includes a shell 602, a faceplate 604, a sensor electrode 606, and a conductive path 608 that electrically connects sensor electrode 606 to a conductive portion 610 of faceplate 604. In addition, configuration 600 includes a push button 612 and a removal handle 614. Push button 612 may be used, for example, to change a hearing program and/or a volume level. Removal handle 614 may assist in removing ITE component 106 from the ear canal of the user. Further, in configuration 600, processor 104 and/or other electronics may be housed within shell 602. Furthermore, in configuration 600, faceplate 604 is customized to the shape of an opening provided in shell 602. As such, faceplate 604 is custom made in any suitable manner, such as described herein, for a particular recipient.

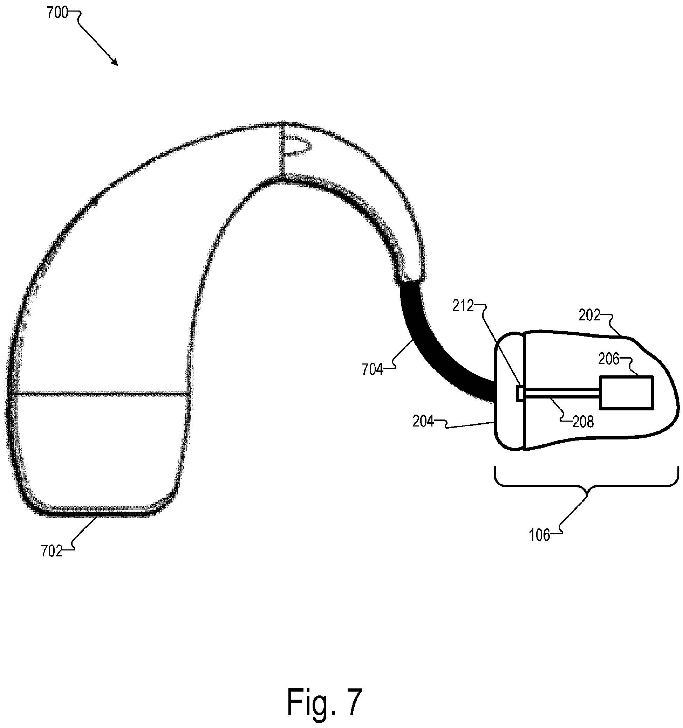

In certain examples, hearing system 100 may further comprise a BTE component that is communicatively coupled to ITE component 106. Such a BTE component is configured to be worn behind an ear of a user and may include circuitry (e.g., a processor similar to processor 104) configured to control operation of a sensor electrode. In such examples, ITE component 106 may further include a receiver (e.g., a speaker) configured to acoustically deliver an audio signal to the user as directed by the BTE component. The BTE component may be communicatively coupled to ITE component 106 in any suitable manner. For example, FIG. 7 illustrates an exemplary configuration 700 in which ITE component 106 is connected to a BTE component 702 by way of a connector portion 704, which may be implemented by any suitable wired connection. In the example shown in FIG. 7, sensor electrode 206 may be electrically connected to circuitry within BTE component 702 by way of conductive path 208, conductive portion 212, and, for example, one or more wires (not shown) that extend within connector portion 704 from faceplate 204 to BTE component 702.

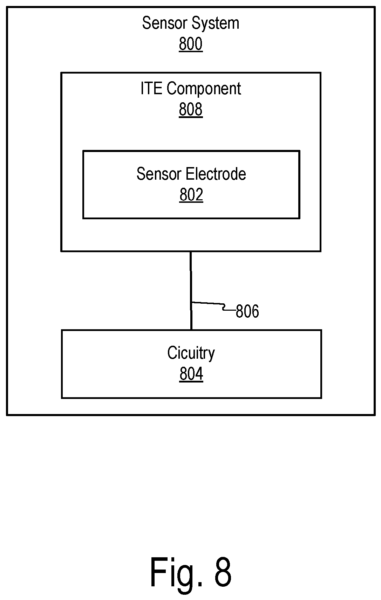

FIG. 8 illustrates an exemplary sensor system 800 that may be implemented in certain examples according to principles described herein. In some instances, sensor system 800 may be implemented by a hearing system comprising an ITE component or a hearing system comprising an ITE component and a BTE component, as described above. As shown in FIG. 8, sensor system 800 includes a sensor electrode 802, circuitry 804, and a conductive path 806. Sensor electrode 802 is configured to be provided on an outer surface of a shell of an ITE component 808 in any suitable manner, such as described herein.

Circuitry 804 may be provided in any suitable location as may serve a particular implementation. For example, circuitry 804 may be provided within a shell of ITE component 808 in certain examples. In such examples, the conductive portion (e.g., conductive portion 212) of the faceplate (e.g., faceplate 204) may be conductively connected to the circuitry housed within the shell. Alternatively, circuitry 804 may be housed within a BTE component (e.g., BTE component 702) that is communicatively coupled to the ITE component. In such examples, the ITE component may further comprise a receiver configured to acoustically deliver an audio signal to the user as directed by the BTE component.

In certain examples, a sensor electrode (e.g., sensor electrode 206) may be conductively connected to circuitry within a shell (e.g., shell 202) by way of a through hole provided in the shell instead of by way of a conductive path that is provided on an outer surface of the shell and that connects to a faceplate (e.g., faceplate 204). In such examples, one or more wires (e.g., litzwires) may be provided through the through hole so as to connect the sensor electrode to electronic components provided within the shell. The one or more wires may be connected in the through hole in any suitable manner. For example, the one or more wires may be connected in the through hole by way of conductive glue, soldering, spring contacts, bolts with a press-fit, etc. In certain examples, the sensor electrode may overlap the through hole provided in the shell. For example, the sensor electrode may be centered over the through hole. In such examples, the sensor electrode may be fixed within the through hole by using glue, any suitable mechanical retention mechanism, and/or a spring.

In certain alternative examples, the through hole may be offset from the sensor electrode on the outer surface of the shell. In such examples, the sensor electrode may be communicatively coupled to one or more wires in the through hole by way of a conductive path that extends along the outer surface of the shell from the sensor electrode to the through hole. When the sensor electrode is offset from the through hole, the sensor electrode and/or conductive path that extends to the through hole may be formed in any suitable manner (e.g., by an LDS process), such as described herein.

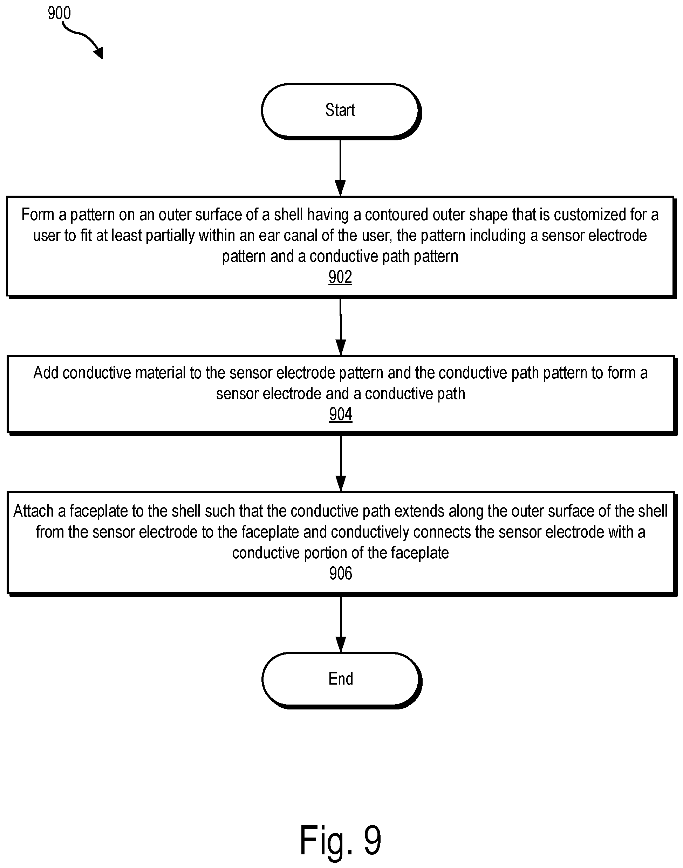

FIG. 9 illustrates an exemplary method for manufacturing a sensor electrode according to principles described herein. While FIG. 9 illustrates exemplary operations according to one embodiment, other embodiments may omit, add to, reorder, and/or modify any of the operations shown in FIG. 9.

In operation 902, a pattern is formed on an outer surface of a shell that has a contoured outer shape that is customized for a user to fit at least partially within an ear canal of the user. The pattern may include a sensor electrode pattern and a conductive path pattern. As described herein, the pattern may be formed by using an LDS process in which a computer-controlled laser forms the pattern and activates an LDS additive included in the shell. Operation 902 may be performed in any of the ways described herein.

In operation 904, conductive material is added to the sensor electrode pattern and the conductive path pattern to form a sensor electrode and a conductive path. As described herein, the conductive material may be added through an electroless plating operation in which one or more metals are added to the sensor electrode pattern and the conductive path pattern. Operation 904 may be performed in any of the ways described herein.

In operation 906, a faceplate is attached to the shell such that the conductive path extends along the outer surface of the shell from the sensor electrode to the faceplate and conductively connects the sensor electrode with a conductive portion of the faceplate. In certain examples, the attaching of the faceplate to the shell may include fitting the faceplate within a custom opening provided in the shell. Alternatively, the attaching of the faceplate to the shell may include fitting a standard faceplate within a standard opening provided in the shell. Operation 906 may be performed in any of the ways described herein.

In certain examples, the method illustrated in FIG. 9 may further include 3D printing the shell such that the shell is customized for the user to fit at least partially within the ear canal of the user.

In the preceding description, various exemplary embodiments have been described with reference to the accompanying drawings. It will, however, be evident that various modifications and changes may be made thereto, and additional embodiments may be implemented, without departing from the scope of the invention as set forth in the claims that follow. The description and drawings are accordingly to be regarded in an illustrative rather than a restrictive sense.

## Claims

1. A hearing system configured to assist a user in hearing, the hearing system comprising:
an in-the-ear (ITE) component (106, 500, 600, 808) comprising:
a shell (202, 602) having a contoured outer shape that is customized for the user to fit at least partially within an ear canal of the user;
a faceplate (204, 604) configured to fit within an opening provided in the shell (202, 602) and face out of the ear canal of the user when the shell (202, 602) is inserted into the ear canal of the user;
a sensor electrode (206, 206 - 1, 206 - 2, 308, 606, 802) that is provided on an outer surface of the shell (202, 602), wherein
the faceplate (204, 604) includes a rim configured to overlap the outer surface of the shell when the faceplate (204, 604) is inserted within the opening in the shell,
**characterized in that**
the in-the-ear component (106, 500, 600, 808) comprises a conductive path (208, 208 - 1, 208 - 2, 310, 608, 806) that extends along the outer surface of the shell (202, 602) from the sensor electrode to the faceplate (204, 604) and that conductively connects the sensor electrode with a conductive portion (212, 212 - 1, 212 - 2, 610) of the faceplate (204, 604), wherein
the conductive portion (212, 212 - 1, 212 - 2, 610) of the faceplate (204, 604) is included on the rim such that, when the faceplate (204, 604) is inserted into the opening in the shell, the conductive portion (212, 212 - 1, 212 - 2, 610) electrically connects circuitry of the hearing system to the sensor electrode by way of the conductive path (208, 208 - 1, 208 - 2, 310, 608, 806).

2. The hearing system of claim 1, wherein the shell (202, 602) is a three-dimensional printed shell that is custom printed for the user to fit at least partially within the ear canal of the user.

3. The hearing system of claim 1 or 2, wherein:
the shell (202, 602) is formed of a material that includes an additive for laser direct structuring (LDS); and
the sensor electrode (206, 206 - 1, 206 - 2, 308, 606, 802) and the conductive path (208, 208 - 1, 208 - 2, 310, 608, 806) are patterned on the outer surface of the shell (202, 602) by an LDS process that activates the additive for LDS.

4. The hearing system of any of the preceding claims, wherein the faceplate (204, 604) is an injection molded faceplate that is formed of a thermoplastic material that includes an additive for laser direct structuring (LDS).

5. The hearing system of any of the preceding claims, wherein the faceplate (204, 604) is a three-dimensional printed faceplate that is custom printed for the user to fit the contoured outer shape of the shell (202, 602).

6. The hearing system of any of the preceding claims, wherein the conductive portion of the faceplate (204, 604) is conductively connected to circuitry housed within the shell (202, 602).

7. The hearing system of any of the preceding claims, further comprising a behind-the-ear (BTE) component (702) that is communicatively coupled to the ITE component (106, 500, 600, 808) and that includes circuitry configured to control operation of the sensor electrode,
wherein the ITE component (106, 500, 600, 808) further comprises a receiver configured to acoustically deliver an audio signal to the user as directed by the BTE component (702).

8. The hearing system of any of the preceding claims, wherein the ITE component (106, 500, 600, 808) further comprises:
a microphone configured to detect an audio signal; and
a receiver configured to deliver the audio signal to the user.

9. The hearing system of any of the preceding claims, wherein the sensor electrode (206, 206 - 1, 206 - 2, 308, 606, 802) is configured to detect a physiological attribute of the user.

10. The hearing system of any of the preceding claims, wherein the conductive path (208, 208 - 1, 208 - 2, 310, 608, 806) is conductively connected to the conductive portion (212, 212 - 1, 212 - 2, 610) of the faceplate (204, 604) by at least one of conductive glue, soldering, spring contacts, and bolts with press-fit.

11. The hearing system of any of the preceding claims, wherein the **ITE** component (106, 500, 600, 808) further comprises:
an additional sensor (206, 206 - 1, 206 - 2, 308, 606, 802) provided on the outer surface of the shell (202, 602); and
an additional conductive path (208, 208 - 1, 208 - 2, 310, 608, 806) that extends along the outer surface of the shell (202, 602) from the additional sensor electrode (206, 206 - 1, 206 - 2, 308, 606, 802) to the faceplate (204, 604) and that conductively connects the additional sensor electrode (206, 206 - 1, 206 - 2, 308, 606, 802) with an additional conductive portion (212, 212 - 1, 212 - 2, 610) of the faceplate (204, 604).

12. The hearing system of any of the preceding claims, further comprising:
circuitry configured to use the sensor electrode (206, 206 - 1, 206 - 2, 308, 606, 802) to detect a physiological attribute of the user.

13. The hearing system of claim 12, wherein:
the circuitry is housed within the shell (202, 602) of the **ITE** component (106, 500, 600, 808); and
the conductive portion (212, 212 - 1, 212 - 2, 610) of the faceplate (204, 604) is conductively connected to the circuitry housed within the shell (202, 602).

14. The hearing system of claim 12, wherein:
the circuitry is housed within a behind-the-ear (BTE) component (702) that is communicatively coupled to the ITE component (106, 500, 600, 808); and
the ITE component (106, 500, 600, 808) further comprises a receiver configured to acoustically deliver an audio signal to the user as directed by the BTE component (702).

## Patentansprüche

1. Hörsystem, das eingerichtet ist, um einen Benutzer beim Hören zu unterstützen, das Hörsystem Folgendes umfassend:
eine Komponente im Ohr (In-the-Ear-, ITE-Komponente) (106, 500, 600, 808), Folgendes umfassend:
eine Ummantelung (202, 602), die eine konturierte äußere Gestalt aufweist, die an den Benutzer angepasst ist, um mindestens teilweise in einen Gehörgang des Benutzers zu passen;
eine Frontplatte (204, 604), die eingerichtet ist, um in eine Öffnung zu passen, die in der Ummantelung (202, 602) bereitgestellt ist, und aus dem Gehörgang des Benutzers herausschaut, wenn die Ummantelung (202, 602) in den Gehörgang des Benutzers eingeführt wird;
eine Sensorelektrode (206, 206-1, 206-2, 308, 606, 802), die auf einer Außenfläche der Ummantelung (202, 602) bereitgestellt ist, wobei
die Frontplatte (204, 604) einen Rand umfasst, der eingerichtet ist, um die Außenfläche der Ummantelung zu überlappen, wenn die Frontplatte (204, 604) in die Öffnung in der Ummantelung eingeführt wird,
**dadurch gekennzeichnet, dass**
die Komponente im Ohr (106, 500, 600, 808) eine Leiterbahn (208, 208-1, 208-2, 310, 608, 806) umfasst, die sich entlang der Außenfläche der Ummantelung (202, 602) von der Sensorelektrode zur Frontplatte (204, 604) erstreckt und die Sensorelektrode mit einem leitfähigen Abschnitt (212, 212-1, 212-2, 610) der Frontplatte (204, 604) leitfähig verbindet, wobei
der leitfähige Abschnitt (212, 212-1, 212-2, 610) der Frontplatte (204, 604) derartig auf dem Rand einbezogen ist, dass, wenn die Frontplatte (204, 604) in die Öffnung in der Ummantelung eingeführt wird, der leitfähige Abschnitt (212, 212-1, 212-2, 610) einen Schaltkomplex des Hörsystems über die Leiterbahn (208, 208-1, 208-2, 310, 608, 806) elektrisch mit der Sensorelektrode verbindet.

2. Hörsystem nach Anspruch 1, wobei die Ummantelung (202, 602) eine dreidimensionale gedruckte Ummantelung ist, die für den Benutzer individuell gedruckt ist, um mindestens teilweise in den Gehörgang des Benutzers zu passen.

3. Hörsystem nach Anspruch 1 bis 2, wobei:
die Ummantelung (202, 602) aus einem Material ausgebildet ist, das einen Zusatzstoff zum Laserdirektstrukturieren (LDS) umfasst; und
die Sensorelektrode (206, 206-1, 206-2, 308, 606, 802) und die Leiterbahn (208, 208-1, 208-2, 310, 608, 806) auf der Außenfläche der Ummantelung (202, 602) durch einen LDS-Prozess strukturiert werden, der den Zusatzstoff für LDS aktiviert.

4. Hörsystem nach einem der vorhergehenden Ansprüche, wobei die Frontplatte (204, 604) eine spritzgegossene Frontplatte ist, die aus einem thermoplastischen Material ausgebildet ist, das einen Zusatzstoff zum Laserdirektstrukturieren (LDS) umfasst.

5. Hörsystem nach einem der vorhergehenden Ansprüche, wobei die Frontplatte (204, 604) eine dreidimensionale gedruckte Frontplatte ist, die für den Benutzer individuell gedruckt ist, um zu der konturierten äußeren Gestalt der Ummantelung (202, 602) zu passen.

6. Hörsystem nach einem der vorhergehenden Ansprüche, wobei der leitfähige Abschnitt der Frontplatte (204, 604) leitfähig mit einem Schaltkomplex verbunden ist, der in der Ummantelung (202, 602) untergebracht ist.

7. Hörsystem nach einem der vorhergehenden Ansprüche, weiterhin eine Komponente hinter dem Ohr (Behind-the-Ear-, BTE-Komponente) (702) umfassend, die kommunikativ mit der ITE-Komponente (106, 500, 600, 808) gekoppelt ist und einen Schaltkomplex umfasst, der eingerichtet ist, um einen Betrieb der Sensorelektrode zu steuern,
wobei die ITE-Komponente (106, 500, 600, 808) weiterhin einen Empfänger umfasst, der eingerichtet ist, um ein Audiosignal akustisch an den Benutzer zu liefern, wie von der BTE-Komponente (702) angewiesen.

8. Hörsystem nach einem der vorhergehenden Ansprüche, wobei die ITE-Komponente (106, 500, 600, 808) weiterhin Folgendes umfasst:
ein Mikrofon, das eingerichtet ist, um ein Audiosignal zu detektieren; und
einen Empfänger, der eingerichtet ist, um das Audiosignal an den Benutzer zu liefern.

9. Hörsystem nach einem der vorhergehenden Ansprüche, wobei die Sensorelektrode (206, 206-1, 206-2, 308, 606, 802) eingerichtet ist, um eine physiologische Eigenschaft des Benutzers zu detektieren.

10. Hörsystem nach einem der vorhergehenden Ansprüche, wobei die Leiterbahn (208, 208-1, 208-2, 310, 608, 806) leitfähig mit dem leitfähigen Abschnitt (212, 212-1, 212-2, 610) der Frontplatte (204, 604) durch mindestens eines von leitfähigem Klebstoff, Löten, Federkontakten und Bolzen mit Presspassung verbunden ist.

11. Hörsystem nach einem der vorhergehenden Ansprüche, wobei die ITE-Komponente (106, 500, 600, 808) weiterhin Folgendes umfasst:
einen zusätzlichen Sensor (206, 206-1, 206-2, 308, 606, 802), der auf der Außenfläche der Ummantelung (202, 602) bereitgestellt ist; und
eine zusätzliche Leiterbahn (208, 208-1, 208-2, 310, 608, 806), die sich entlang der Außenfläche der Ummantelung (202, 602) von der zusätzlichen Sensorelektrode (206, 206-1, 206-2, 308, 606, 802) zur Frontplatte (204, 604) erstreckt und die zusätzliche Sensorelektrode (206, 206-1, 206-2, 308, 606, 802) mit einem zusätzlichen leitfähigen Abschnitt (212, 212-1, 212-2, 610) der Frontplatte (204, 604) leitfähig verbindet.

12. Hörsystem nach einem der vorhergehenden Ansprüche, weiterhin Folgendes umfassend
einen Schaltkomplex, der eingerichtet ist, um die Sensorelektrode (206, 206-1, 206-2, 308, 606, 802) zu verwenden, um eine physiologische Eigenschaft des Benutzers zu detektieren.

13. Hörsystem nach Anspruch 12, wobei:
der Schaltkomplex innerhalb der Ummantelung (202, 602) der ITE-Komponente (106, 500, 600, 808) untergebracht ist; und
der leitfähige Abschnitt (212, 212-1, 212-2, 610) der Frontplatte (204, 604) leitfähig mit dem Schaltkomplex verbunden ist, der innerhalb der Ummantelung (202, 602) untergebracht ist.

14. Hörsystem nach Anspruch 12, wobei:
der Schaltkomplex innerhalb einer Komponente hinter dem Ohr (Behind-the-Ear-, BTE-Komponente) (702) untergebracht ist, die kommunikativ mit der ITE-Komponente (106, 500, 600, 808) gekoppelt ist; und
die ITE-Komponente (106, 500, 600, 808) weiterhin einen Empfänger umfasst, der eingerichtet ist, um ein Audiosignal akustisch an den Benutzer zu liefern, wie von der BTE-Komponente (702) angewiesen.

## Revendications

1. Système auditif configuré pour aider un utilisateur à entendre, le système auditif comprenant :
un composant intra-auriculaire (ITE) (106, 500, 600, 808) comprenant :
une coque (202, 602) ayant une forme extérieure profilée qui est personnalisée pour l'utilisateur afin de s'adapter au moins partiellement dans un conduit auditif de l'utilisateur ;
une plaque frontale (204, 604) configurée pour s'adapter dans une ouverture prévue dans la coque (202, 602) et faire face à l'extérieur du conduit auditif de l'utilisateur lorsque la coque (202, 602) est insérée dans le conduit auditif de l'utilisateur ;
une électrode de capteur (206, 206-1, 206-2, 308, 606, 802) qui est prévue sur une surface extérieure de la coque (202, 602),
la plaque frontale (204, 604) comprenant un rebord configuré pour chevaucher la surface extérieure de la coque lorsque la plaque frontale (204, 604) est insérée dans l'ouverture de la coque,
**caractérisé en ce que**
le composant intra-auriculaire (106, 500, 600, 808) comprend un chemin conducteur (208, 208-1, 208-2, 310, 608, 806) qui s'étend le long de la surface extérieure de la coque (202, 602) depuis l'électrode de capteur jusqu'à la plaque frontale (204, 604) et qui connecte de manière conductrice l'électrode de capteur avec une partie conductrice (212, 212-1, 212-2, 610) de la plaque frontale (204, 604),
la partie conductrice (212, 212-1, 212-2, 610) de la plaque frontale (204, 604) étant incluse sur le rebord de sorte que, lorsque la plaque frontale (204, 604) est insérée dans l'ouverture de la coque, la partie conductrice (212, 212-1, 212-2, 610) connecte électriquement des circuits du système auditif à l'électrode de capteur par l'intermédiaire du chemin conducteur (208, 208-1, 208-2, 310, 608, 806).

2. Système auditif selon la revendication 1, la coque (202, 602) étant une coque imprimée en trois dimensions qui est imprimée sur mesure pour l'utilisateur afin de s'adapter au moins partiellement dans le conduit auditif de l'utilisateur.

3. Système auditif selon la revendication 1 à 2 :
la coque (202, 602) étant formée d'un matériau qui comprend un additif pour structuration directe par laser (LDS) ; et
l'électrode de capteur (206, 206-1, 206-2, 308, 606, 802) et le chemin conducteur (208, 208-1, 208-2, 310, 608, 806) étant modelés sur la surface extérieure de la coque (202, 602) par un procédé LDS qui active l'additif pour LDS.

4. Système auditif selon l'une quelconque des revendications précédentes, la plaque frontale (204, 604) étant une plaque frontale moulée par injection qui est formée d'un matériau thermoplastique qui comprend un additif pour structuration directe par laser (LDS).

5. Système auditif selon l'une quelconque des revendications précédentes, la plaque frontale (204, 604) étant une plaque frontale imprimée en trois dimensions qui est imprimée sur mesure pour l'utilisateur afin de s'adapter à la forme extérieure profilée de la coque (202, 602).

6. Système auditif selon l'une quelconque des revendications précédentes, la partie conductrice de la plaque frontale (204, 604) étant connectée de manière conductrice à des circuits logés à l'intérieur de la coque (202, 602).

7. Système auditif selon l'une quelconque des revendications précédentes, comprenant en outre un composant contour d'oreille (BTE) (702) qui est couplé de manière communicative au composant ITE (106, 500, 600, 808) et qui comprend des circuits configurés pour contrôler le fonctionnement de l'électrode de capteur, le composant ITE (106, 500, 600, 808) comprenant en outre un récepteur configuré pour délivrer acoustiquement un signal audio à l'utilisateur tel que dirigé par le composant BTE (702).

8. Système auditif selon l'une quelconque des revendications précédentes, le composant ITE (106, 500, 600, 808) comprenant en outre :
un microphone configuré pour détecter un signal audio ; et
un récepteur configuré pour délivrer le signal audio à l'utilisateur.

9. Système auditif selon l'une quelconque des revendications précédentes, l'électrode de capteur (206, 206-1, 206-2, 308, 606, 802) étant configurée pour détecter un attribut physiologique de l'utilisateur.

10. Système auditif selon l'une quelconque des revendications précédentes, le chemin conducteur (208, 208-1, 208-2, 310, 608, 806) étant connecté de manière conductrice à la partie conductrice (212, 212-1, 212-2, 610) de la plaque frontale (204, 604) par au moins l'un parmi une colle conductrice, une soudure, des contacts à ressort, et des boulons avec ajustement serré.

11. Système auditif selon l'une quelconque des revendications précédentes, le composant ITE (106, 500, 600, 808) comprenant en outre :
un capteur supplémentaire (206, 206-1, 206-2, 308, 606, 802) prévu sur la surface extérieure de la coque (202, 602) ; et
un chemin conducteur supplémentaire (208, 208-1, 208-2, 310, 608, 806) qui s'étend le long de la surface extérieure de la coque (202, 602) depuis l'électrode de capteur supplémentaire (206, 206-1, 206-2, 308, 606, 802) jusqu'à la plaque frontale (204, 604) et qui connecte de manière conductrice l'électrode de capteur supplémentaire (206, 206-1, 206-2, 308, 606, 802) avec une partie conductrice supplémentaire (212, 212-1, 212-2, 610) de la plaque frontale (204, 604).

12. Système auditif selon l'une quelconque des revendications précédentes, comprenant en outre :
des circuits configurés pour utiliser l'électrode de capteur (206, 206-1, 206-2, 308, 606, 802) afin de détecter un attribut physiologique de l'utilisateur.

13. Système auditif selon la revendication 12 :
les circuits étant logés à l'intérieur de la coque (202, 602) du composant ITE (106, 500, 600, 808) ; et
la partie conductrice (212, 212-1, 212-2, 610) de la plaque frontale (204, 604) étant connectée de manière conductrice aux circuits logés à l'intérieur de la coque (202, 602).

14. Système auditif selon la revendication 12 :
les circuits étant logés à l'intérieur d'un composant contour d'oreille (BTE) (702) qui est couplé de manière communicative au composant ITE (106, 500, 600, 808) ; et
le composant ITE (106, 500, 600, 808) comprenant en outre un récepteur configuré pour délivrer acoustiquement un signal audio à l'utilisateur tel que dirigé par le composant BTE (702).
